# FASCICULE DE BREVET EUROPEEN

(11) **EP 2 516 374 B1**
(45) Date de publication et mention de la délivrance du brevet: **17.02.2016**
(21) Numéro de dépôt: 10805466.9
(22) Date de dépôt: 07.12.2010
(51) Int. Cl.: C07C 45/52, C07C 45/75, C07C 47/22

(54) **PROCEDE DE SYNTHESE PERFECTIONNE D'ACROLEINE A PARTIR DE GLYCEROL**
VERBESSERTES VERFAHREN ZUR SYNTHESE VON ACROLEIN AUS GLYCERIN
IMPROVED METHOD FOR SYNTHESIZING ACROLEIN FROM GLYCEROL

(30) Priorité: 22.12.2009 FR 0959379
(43) Date de publication de la demande: 31.10.2012
(73) Titulaire: Arkema France, 92700 Colombes (FR)
(72) Inventeur: DUBOIS, Jean-Luc, F-69390 Millery (FR)
(74) Mandataire: Bonnel, Claudine
(86) Numéro de dépôt international: PCT/FR2010/052626
(87) Numéro de publication internationale: WO 2011/083225

(56) Documents cités:
- FR-A1- 2 230 613
- CORMA A ET AL: "Biomass to chemicals: Catalytic conversion of glycerol/water mixtures into acrolein, reaction network", JOURNAL OF CATALYSIS, ACADEMIC PRESS, DULUTH, MN, US LNKD- DOI:10.1016/J.JCAT.2008.04.016, vol. 257, no. 1, 1 juillet 2008 (2008-07-01), pages 163-171, XP022710527, ISSN: 0021-9517 [extrait le 2008-05-23]

## Description

La présente invention a pour objet un procédé de synthèse d'acroléine couplant une déshydratation du glycérol en acroléine avec une réaction d'aldolisation de l'acétaldéhyde, produit à l'état d'impureté lors de la déshydratation, par du formaldéhyde.

L'acroléine est le nom usuel donné à l'aldéhyde insaturé de formule CH₂=CH-CHO appelé propénal. Ce composé est connu depuis fort longtemps et est utilisé comme biocide à large spectre ; il est également un intermédiaire pour la synthèse de divers produits tels que la D,L-Methionine (supplément pour nourriture animale), de l'acide acrylique, des produits pharmaceutiques, des fongicides, des parfums, de la pyridine, des picolines, du glutaraldéhyde...

Les grands procédés industriels de fabrication de l'acroléine sont aujourd'hui celui développé par la société DEGUSSA au cours des années 1940 utilisant comme matières premières l'acétaldéhyde et le formol, et celui mis en place quelque 20 ans plus tard par diverses sociétés consistant à oxyder le propylène.

Le procédé DEGUSSA conduit en phase gazeuse par catalyse hétérogène est fondé sur la réaction suivante :

CH₃-CHO + CH₂O → CH₂=CH-CHO + H₂O.

Le second procédé qui a supplanté industriellement le précédent est fondé sur la réaction d'oxydation suivante :

CH₂=CH-CH₃ + O₂ → CH₂=CH-CHO + H₂O.

Plus récemment, des travaux importants ont été réalisés pour faire évoluer un autre procédé de synthèse à partir de glycérol soumis à une déshydratation selon la réaction suivante :

CH₂OH-CHOH-CH₂OH → CH₂=CH-CHO + 2H₂O

Ce type de procédé présente l'immense avantage de pouvoir travailler avec une matière première naturelle non fossile et donc de répondre en produisant ce qu'on appelle une acroléine bio-ressourcée, aux engagements de la plupart des pays industrialisés visant à réduire les émissions de gaz à effet de serre avec ses effets environnementaux.

On entend par acroléine « bio-ressourcée » un composé présentant une teneur en carbone ¹⁴C caractéristique de l'origine naturelle non fossile des matières premières utilisées.

L'utilisation de matières premières carbonées d'origine naturelle et renouvelable peut se détecter grâce aux atomes de carbone entrant dans la composition du produit final. En effet, à la différence des matériaux issus de matières fossiles, les matériaux composés de matières premières renouvelables contiennent du ¹⁴C. Tous les échantillons de carbone tirés d'organismes vivants (animaux ou végétaux) sont en fait un mélange de 3 isotopes : ¹²C (représentant ∼ 98,892 %), ¹³C (∼ 1,108 %) et ¹⁴C (traces: 1,2.10⁻¹⁰ %). Le rapport ¹⁴C /¹²C des tissus vivants est identique à celui de l'atmosphère. Dans l'environnement, le ¹⁴C existe sous deux formes prépondérantes : sous forme minérale c'est-à-dire de gaz carbonique (CO₂) et sous forme organique c'est à dire de carbone intégré dans des molécules organiques.

Dans un organisme vivant, le rapport ¹⁴C /¹²C est maintenu constant par le métabolisme car le carbone est continuellement échangé avec l'environnement. La proportion de ¹⁴C étant sensiblement constante dans l'atmosphère, il en est de même dans l'organisme, tant qu'il est vivant, puisqu'il absorbe ce ¹⁴C comme il absorbe le ¹²C. Le rapport moyen de ¹⁴C/¹²C est égal à 1,2x10⁻¹².

Le ¹²C est stable, c'est-à-dire que le nombre d'atomes de ¹²C dans un échantillon donné est constant au cours du temps. Le ¹⁴C, lui, est radioactif et chaque gramme de carbone d'être vivant contient suffisamment d'isotope ¹⁴C pour donner 13,6 désintégrations par minute.

La demi-vie (ou période) T_{1/2}, liée à la constante de désintégration du ¹⁴C est de 5730 ans. Compte tenu de cette durée, on considère que la teneur en ¹⁴C est pratiquement constante depuis l'extraction des matières premières végétales jusqu'à la fabrication du produit final.

A l'heure actuelle, il existe au moins deux techniques différentes pour la mesure de la teneur en ¹⁴C d'un échantillon :
- par spectrométrie à scintillation liquide
- par spectrométrie de masse : l'échantillon est réduit en graphite ou en CO₂ gazeux, analysé dans un spectromètre de masse. Cette technique utilise un accélérateur et un spectromètre de masse pour séparer les ions ¹⁴C des ¹²C et donc déterminer le rapport des deux isotopes.

Ces méthodes de mesure de la teneur en ¹⁴C des matériaux sont décrites précisément dans les normes ASTM D 6866 (notamment D6866-06) et dans les normes ASTM D 7026 (notamment 7026-04). La méthode de mesure préférentiellement utilisée est la spectrométrie de masse décrite dans la norme ASTM D6866-06 (" accelerator mass spectroscopy ").

L'invention a pour objet un procédé de synthèse d'acroléine « bio-ressourcée » ayant une teneur en masse de ¹⁴C telle que le ratio ¹⁴C/¹²C est compris entre 0,2.10⁻¹² et 1,2.10⁻¹². De préférence le ratio ¹⁴C/¹²C est compris entre 0,6.10⁻¹² et 1,2.10⁻¹² et de façon plus préférée entre 0,9.10⁻¹² et 1,2.10⁻¹².

Le ratio ¹⁴C/¹²C dépendra des modes de fabrication mis en oeuvre, des matières premières utilisées, toutes ou partiellement d'origine naturelle non fossile, ou en fonction de mélanges ultérieurement effectués. Ce ratio ne peut pas dépasser 1,2.10⁻¹²; si tel était le cas cela impliquerait que l'opérateur a introduit artificiellement les atomes ¹⁴C dans le composé acroléine.

Les procédés connus de production d'acroléine bioressourcée à partir de glycérol conduisent cependant à des rendements qui restent globalement insuffisants. Ces rendements faibles s'expliquent en partie par la présence dans l'effluent sortant du réacteur de quantités importantes de sous-produits comme l'acétaldéhyde et le formaldéhyde, issus de la décomposition du glycérol, de l'acroléine ou d'autres intermédiaires. Une part substantielle de la matière première, de l'ordre de 5 à 10 %, peut ainsi être perdue sous la forme de sous-produits. Dans l'article J. Catal., 257, 163-171 (2008), A. Corma et al suggèrent de séparer et isoler l'acétaldéhyde pour le valoriser commercialement.

L'invention a pour but de pallier les inconvénients précédents en proposant un procédé de synthèse d'acroléine « bio-ressourcée » utilisant principalement des matières premières naturelles non fossiles et offrant un rendement supérieur.

La présente invention a donc pour objet un procédé de synthèse d'acroléine à partir de glycérol, offrant le meilleur rendement possible en acroléine, mais aussi la meilleure façon de valoriser les sous-produits, généralement perdus à hauteur de 5 à 10% de la matière première, notamment la meilleure valorisation possible de l'acétaldéhyde contenu dans le flux d'acroléine issu de la réaction de déshydratation du glycérol.

Le procédé de synthèse d'acroléine selon l'invention consiste
- dans une première étape à soumettre une charge de glycérol issue de la méthanolyse des huiles végétales ou des graisses animales, à une réaction de déshydratation conduisant à l'acroléine selon la réaction CH₂OH-CHOH-CH₂OH → CH₂=CH-CHO + 2H₂O puis,
- dans une deuxième étape après avoir soumis l'effluent issu de la première étape à un refroidissement à procéder tout d'abord dans une première zone à un lavage à l'eau et à la condensation de l'effluent gazeux issu de la première étape, pour séparer ensuite dans une deuxième zone de fractionnement d'une première part un flux riche en aldéhydes légers, d'une deuxième part un flux riche en eau et d'une troisième part le flux d'acroléine, puis
- dans une troisième étape à faire réagir l'acétaldéhyde contenu dans le flux riche en aldéhydes légers issu de l'étape précédente avec du formaldéhyde pour obtenir un second flux riche en acroléine par réaction d'aldolisation selon le schéma réactionnel suivant : CH₃-CHO + CH₂O → CH₂=CH-CHO + H₂O et à recycler ce second flux riche en acroléine au niveau de l'étape 2 précédente, le réacteur d'aldolisation étant alimenté par le flux d'aldéhydes légers issu de l'étape 2 qui est complété par un flux auxiliaire d 'acétaldéhyde et/ou de formaldéhyde tel que le rapport molaire acétaldéhyde/formaldéhyde au sein du réacteur est comprise entre 0,3 et 1,5.

Bien que la réaction d'aldolisation entre l'acétaldéhyde et le formaldéhyde pour produire de l'acroléine soit connue de l'art antérieur, aucun document ne suggère les avantages de la présente invention liés à l'association de cette réaction avec un procédé de synthèse d'acroléine à partir de glycérol.

La charge utilisée dans la 1^{ère} étape du premier procédé est le glycérol - 1,2,3-propane triol - qui est un co-produit formé lors de la méthanolyse ou plus généralement des alcoolyses, hydrolyses et saponifications, des huiles végétales ou des graisses animales, l'autre co-produit étant les esters méthyliques qui sont employés notamment comme gazole et fioul domestique, ou les acides gras (hydrolyse) ou les savons (saponification). Le développement des « biocarburants » entraîne une augmentation de la production de glycérol selon cette voie où le glycérol représente de l'ordre de 10 % du poids de l'huile transformée.

Le glycérol pourra être au préalable soumis à différents traitements de purification visant l'élimination des sels par distillation, par utilisation de résines échangeuses d'ions ou par utilisation d'un lit fluidisé (demande de brevet français 2 913 974) ou la purification et l'évaporation du glycérol, notamment décrites par G.B. D'Souza, dans J. Am. Oil Chemists' Soc. November 1979 (Vol 56) 812A, par Steinberner U et al., dans Fat. Sci. Technol. (1987), 89 Jahrgang Nr.8, pp 297-303, et par Anderson D.D. et al. dans Soaps and Detergents : A theoretical and Practical Review, Miami Beach Fla., Oct 12-14 1994, chapter 6 pp172-206. Ed: L Spitz, AOCS Press, Champaign.

On utilise généralement des solutions aqueuses de glycérol dont la concentration peut varier dans de larges mesures, par exemple de 20 à 99% en poids de glycérol, de préférence on utilise des solutions comportant de 30 à 80% en poids de glycérol.

La réaction de déshydratation CH₂OH-CHOH-CH₂OH ↔ CH₂OH-CH₂-CHO + H₂O → CH₂=CH-CHO + 2H₂O est une réaction équilibrée qui est favorisée par un niveau de température élevé. Elle est effectuée en général en phase gaz dans le réacteur en présence d'un catalyseur solide à une température allant de 150°C à 500°C, de préférence comprise entre 250°C et 350°C, et une pression comprise entre 1 et 5 bars. Elle peut également être conduite en phase liquide ainsi que cela est décrit dans le brevet US 5,387,720 colonne 3 ligne 13 à colonne 4 ligne 11. On peut aussi l'effectuer en présence d'oxygène ou d'un gaz contenant de l'oxygène comme décrit dans les demandes WO 06/087083 et WO 06/114506. Dans ces procédés, l'oxygène n'a pas une fonction d'oxydation mais contribue en fait à décoker le catalyseur, en brûlant le coke formé. Ce catalyseur n'est pas oxydant, dans le sens où il forme peu d'acide acrylique et d'acide acétique, mais acide, et il s'encrasse rapidement, et son décokage permet donc de prolonger sa durée de vie ; en outre, l'oxygène contribue aussi à diminuer la formation de sous-produits gênants comme le phénol, l'acétone et le propanaldéhyde par exemple. Dès lors que la température n'est pas trop élevée, il n'y a pas d'oxydation en acide acrylique.

La réaction de déshydratation du glycérol est généralement effectuée sur des catalyseurs solides acides. Les catalyseurs qui conviennent sont des matériaux homogènes ou multiphases, insolubles dans le milieu réactionnel qui ont une acidité de Hammett, notée H₀ inférieure à +2.

Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse.

Ces catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides.

Ces catalyseurs pourront généralement être constitués par un sel d'hétéropolyacide dans lequel des protons dudit hétéropolyacide sont échangés avec au moins un cation choisi parmi les éléments appartenant aux Groupes I à XVI de la Classification Périodique des Eléments, ces sels d'hétéropolyacide contenant au moins un élément choisi parmi le groupe comprenant W, Mo et V.

Parmi les oxydes mixtes, on peut citer particulièrement ceux à base de fer et de phosphore, ceux à base de Vanadium et Phosphore, ceux à base d'Aluminium et Phosphore, Bore et Phosphore, Phosphore ou silicium et tungstène et ceux à base de césium, phosphore et tungstène.

Les catalyseurs sont notamment choisis parmi les zéolithes, les composites Nafion^{®} (à base d'acide sulfonique de polymères fluorés), les alumines chlorées, les acides et sels d'acides phosphotungstiques et/ou silicotungstiques, et différents solides de type oxydes métalliques tels que oxyde de tantale Ta₂O₅, oxyde de niobium Nb₂O₅, alumine Al₂O₃, oxyde de titane TiO₂, zircone ZrO₂, oxyde d'étain SnO₂, silice SiO₂ ou silico-aluminate SiO₂-Al₂O₃, imprégnés de fonctions acides telles que borate BO₃, sulfate SO₄, tungstate WO₃, phosphate PO₄, silicate SiO₂ ou molybdate MoO₃ ou un mélange de ces composés.

Les catalyseurs précédents peuvent comprendre en plus un promoteur tel que Au, Ag, Cu, Pt, Rh, Pd, Ru, Sm, Ce, Yt, Sc, La, Zn, Mg, Fe, Co, Ni.

Les catalyseurs préférés sont les zircones phosphatées, les zircones tungstées, les zircones silicées, les oxydes de titane ou d'étain imprégnés de tungstate, silicotungstate ou phosphotungstate, les alumines ou silices phosphatées, les hétéropolyacides ou sels d'hétéropolyacides, les phosphates de fer et les phosphates de fer comprenant un promoteur, les oxydes mixtes Vanadium-Phosphore.

Dans le cas d'une mise en oeuvre de la réaction en phase liquide, les catalyseurs utilisés, de même nature que les précédents devront eux présenter une acidité de Hammett H₀ généralement inférieure à +2, de préférence comprise entre - 20 et - 8,2. Cette forme de mise en oeuvre de la réaction décrite dans la publication de Benjamin Katryniok et al. « Towards the Sustainable Production of Acrolein by Glycerol Dehydration » paru dans ChemSusChem 2009 ,2,719-730 Ed. Wiley, pose cependant des problèmes au niveau industriel en raison des conditions de pression fort élevées et d'autre part de la sélectivité de la réaction dès lors que l'on cherche à augmenter le taux de conversion. C'est la raison pour laquelle le procédé en phase liquide n'est pas jusqu'à maintenant adopté par les industriels ; il faut cependant noter que le procédé de l'invention permet de résoudre le problème posé par la sélectivité et donc d'améliorer sensiblement les performances d'un procédé en phase liquide.

La deuxième étape est conduite dans un ensemble constitué d'une zone de condensation généralement dénommée absorption/désorption comportant au moins deux colonnes, la première permettant d'éliminer les composés lourds et la deuxième la désorption des composés incondensables. Par colonne il faut entendre colonne mais également échangeur de chaleur, condenseur ou de tout système équivalent. L'ensemble comprend également une zone de fractionnement comportant au moins une colonne de distillation permettant la séparation d'une part de la fraction aqueuse de la solution et d'autre part du flux riche en acroléine et du flux plus léger enrichi en aldéhydes légers tels que par exemple l'acétaldéhyde.

L'effluent gazeux issu du réacteur de déshydratation est soumis, à la sortie de celui-ci, à un refroidissement de type « quench » dans tout dispositif convenable tel qu'une « chaudière gaz - échangeur de chaleur » permettant la production de vapeur, puis envoyé au sein de la zone de condensation où lui est appliqué un lavage à l'eau permettant l'absorption de l'essentiel des constituants de l'effluent gazeux, dans une colonne d'absorption/condensation fonctionnant, à une température comprise généralement entre 0 et 90 °C, la température des gaz en tête de colonne étant de préférence comprise entre 30 et 60 °C et celle du liquide de fond de colonne comprise entre 60 et 90 °C. Dans cette colonne, l'acide acrylique, l'acide acétique, mais aussi les polyéthers de glycérol, les acétals de glycérol, le glycérol résiduel... sont séparés en fond, alors que l'acroléine, l'acétaldéhyde, le formaldéhyde, l'acétone, le propionaldéhyde et les gaz n'ayant pas réagi O₂, N₂... ainsi que CO, CO₂... sont dégagés en tête de colonne. L'effluent de tête de cette première colonne est envoyé vers une deuxième colonne d'élimination des incondensables (gaz légers), en fond de laquelle on recueille une solution aqueuse d'acroléine contenant les composés légers solubles dans les conditions, les composés incondensables étant purgés en tête de colonne. La solution aqueuse d'acroléine est adressée à la zone de fractionnement par distillation pour séparer une fraction d'aldéhydes légers contenant l'acétaldéhyde ainsi que d'autres composés légers tels que le formaldéhyde et une fraction plus lourde contenant l'acroléine ainsi que souvent du propanaldéhyde et de l'acétone en solution dans l'eau, fraction plus lourde dont le solvant aqueux qui, extrait après séparation par distillation, peut être recyclé.

Dans une variante de mise en oeuvre de la zone de condensation, la colonne d'absorption sera couplée avec une autre colonne à laquelle sera adressé l'effluent de queue de la colonne d'absorption/condensation pour permettre une meilleure séparation des composés lourds et une récupération des composés plus légers ayant été entraînés dans cette fraction lourde.

Pour ce qui concerne la zone de fractionnement celle-ci comportera généralement au moins deux colonnes de distillation successives; en tête de la première colonne le flux riche en aldéhydes légers, acétaldéhyde et autres composés légers sera extrait et l'effluent liquide de queue de cette première colonne sera transféré dans une deuxième colonne dans laquelle on séparera en tête le flux d'acroléine et en queue l'effluent aqueux.

Dans une variante de mise en oeuvre de cette zone de fractionnement on réalisera la séparation de l'acroléine, des aldéhydes légers et de l'eau dans une seule colonne avec un soutirage latéral comme décrit dans le brevet US 6,515,187. Ce type de colonnes « compactées » permet de limiter les investissements. Elles sont aussi illustrées par les schémas figurant dans les Techniques de l'Ingénieur J6100 page 2 et dans les encyclopédies Ullmann et Kirk Othmer page 154 et 287 par exemple.

La fraction légère recueillie en tête de colonne de distillation riche en aldéhydes légers tels que l'acétaldéhyde et le formaldéhyde est adressée à la troisième étape éventuellement après des traitements complémentaires de purification.

La composition de cette fraction légère riche en aldéhydes légers dépend des conditions opératoires de la réaction de déshydratation ainsi que de celles des phases d'absorption/désorption. En général, la teneur en acétaldéhyde est sensiblement plus élevée que celle de formaldéhyde. Cependant cela ne signifie pas que systématiquement la teneur en acétaldéhyde sera supérieure à celle du formaldéhyde dans cette fraction légère riche en aldéhydes légers. En résumé, cette fraction légère contiendra toujours de l'acétaldéhyde et une quantité variable de formaldéhyde fonction des conditions opératoires.

Lors de la troisième étape, on fait réagir l'acétaldéhyde issu de l'étape précédente avec du formaldéhyde selon une réaction d'aldolisation dont le schéma réactionnel est décrit ci-dessous. L'acétaldéhyde sera introduit dans le réacteur d'aldolisation via le flux constituant la fraction légère riche en aldéhydes légers issue de l'étape 2 riche en acétaldéhyde. Le formaldéhyde sera introduit dans le réacteur d'aldolisation d'une part via le flux constituant la fraction légère riche en aldéhydes légers et d'autre part sous forme de formaldéhyde frais. Dans le cas où la teneur en formaldéhyde de la fraction légère riche en aldéhydes légers serait telle que le ratio formaldéhyde/acétaldéhyde serait supérieur à celui fixé pour la conduite de la réaction, de l'acétaldéhyde frais serait introduit dans le réacteur d'aldolisation pour atteindre ledit ratio. On obtient ainsi par réaction d'aldolisation un flux supplémentaire d'acroléine. Ce flux supplémentaire est ensuite recyclé au niveau de l'étape 2. Cette réaction est réalisée en phase gazeuse à une température comprise entre 150 et 400 °C de préférence entre 260 et 350°C à une vitesse spatiale horaire (VVH) de 100 à 2500 h⁻¹, à une pression généralement comprise entre 0,5 et 10 bars, de préférence entre 0,8 et 2 bars, en présence d'un catalyseur solide de condensation. Un inhibiteur de polymérisation tel que la phénothiazine, l'hydroquinone, l'éther monométhylique d'hydroquinone, le diterbutyl para-crésol (BHT), la paraphénylène diamine ou un dérivé du Tempo peut être ajouté dans le réacteur d'aldolisation.

Les catalyseurs de condensation solides utilisables dans le procédé de l'invention sont généralement des oxydes naturels ou synthétiques (zéolithes par exemple) dont la gamme est très large mais devant présenter certaines caractéristiques. En effet la réaction de condensation (aldolisation) est en fait une réaction en deux étapes au cours de laquelle on forme tout d'abord un aldéhyde hydroxylé qui conduit ensuite à l'aldéhyde insaturé par déshydratation. La réalisation de ces deux étapes nécessite un catalyseur ayant des sites acides mais devant présenter également de préférence des sites basiques afin de mener à bien l'ensemble des réactions.

Le choix des meilleurs catalyseurs s'effectuera sur la base de deux critères liés à l'adsorption de molécules test: NH₃ (acidité), CO₂ et SO₂ (basicité).

Le premier critère est la chaleur moyenne d'adsorption de ces molécules test sur ledit solide. La méthode de mesure par microcalorimétrie différentielle est décrite dans le Handbook of Thermal Analysis and Calorimetry ; vol. 5 Chapter 11, pages 387-438 « Heterogeneous Catalysis on Solids ».

La chaleur moyenne d'adsorption du CO₂ devra être comprise entre 40 kJ/mole et 170 kJ/mole à 303 °K.

La chaleur moyenne d'adsorption du SO₂ devra être comprise entre 25 kJ/mole et 180 kJ/mole à 353 °K.

La chaleur moyenne d'adsorption de NH₃ devra être comprise entre 25 kJ/mole et 170 kJ/mole à 423 °K.

Le deuxième critère est la quantité adsorbée de ces molécules test par unité de surface de ces solides tel que décrit dans l'article « Microcalorimetric Study of the Acidity and Basicity of Metal Oxide Surfaces » de Aline Auroux et Antonella Gervasini paru dans J. Phys. Chem. 1990, 94, 6371-6379.

La quantité de NH₃ adsorbée sur la surface du catalyseur sera comprise entre 1,5 et 8 micromole/m² et celle de CO₂ comprise entre 0,5 et 8 micromole/m².

A titre d'exemples de constituants du catalyseur solide, on peut citer : BaO, SrO, CaO, MgO, ZnO, TiO₂, ZrO₂ sous forme oxyde ou carbonate, les oxydes de type argile comme les hydrotalcites, chrysotile et sépiolite, les zéolithes échangées avec des ions alcalins (Cs, K, Na, Li), les fluorures alcalins (tels que KF par exemple) déposés sur alumine, des oxydes ou oxycarbonates de terres rares, les solides de type métaux alcalins supportés sur alumine (tel que Na/Al₂O₃) ou sur magnésie (tels que Li/MgO) ou les oxydes de terres rares dopées par des alcalinao-terreux tels que (SrO-La₂O₃).

Ce catalyseur solide sera constitué par exemple de silicate de sodium sur silice, ou d'une silice-alumine présentant de préférence un rapport atomique Si/Al supérieur à 10, comportant le cas échéant un promoteur. On peut citer aussi à titre d'exemple de tels catalyseurs, les aluminosilicates cristallisés ou amorphes, les silicalites, les zéolithes synthétiques cristallines telles que la Faujasite, la Ferriérite, la ZSM-5, sous leur forme acide ou soit partiellement, soit totalement neutralisée par des éléments des groupes 1 à 14, et de préférence des groupes 1 et 2 et par Zn et Tl. Les zéolithes utilisées peuvent avoir dans leur structure une partie ou la totalité des atomes d'aluminium remplacés par des atomes trivalents tels que B, Ga, Fe, Cr, V, As, Sb, Bi, Be, et peuvent avoir une partie ou la totalité des atomes de silicium remplacés par des atomes tétravalents tels que Ge, Ti, Zr, Hf.

D'autres catalyseurs peuvent aussi convenir pour cette réaction tels que les catalyseurs oxydes mixtes de type phosphates mixtes de Cobalt et Aluminium, Alumine, Silice ou Silice-Alumine dopées par exemple avec des sels de sodium (Na), potassium (K), Cesium (Cs), cadmium (Cd), Mg, Ca, Sr, Mn, Zn, Mo, Nb, Pb, Si. Ils pourront être MgO-Alumine, MgO-SiO₂...terres rares, sous forme de phosphates, tungstates, molybdates..... Les hydrotalcites, les hydroxyapatites, les oxynitrures de dérivés phosphorés tels que les oxynitrures mixtes de Vanadium-Aluminium, Phosphore-Zirconium, Phosphore-Aluminium, Vanadium-Aluminium-Phosphore, Gallium-Aluminium-Phosphore peuvent aussi convenir pour cette réaction. De même pourront être utilisés les solides basiques tels que définis dans la table 1.2 Solid Bases de la publication « Définition and Classification of Solid Acids and Bases, Their Catalytic Properties » , de l'ouvrage « New Solid Acids and Bases », de Tanabe et al., Kodansha, Tokyo, 1989 pages 1 à 3, et pages 326 à 329.

Le facteur le plus important pour orienter la sélectivité vers telle ou telle molécule réside dans la nature et quantités respectives des réactifs mis enjeu. On ajoute au milieu réactionnel du formaldéhyde en quantité telle que rapport molaire formaldéhyde/ acétaldéhyde est compris entre 0,3 et 1,5 , de préférence entre 0,5 et 1,0.

La réaction est de préférence réalisée en présence d'oxygène ou d'air qui permettra d'éviter une désactivation rapide du catalyseur. Celui-ci peut cependant être régénéré par traitement à l'air à une température comprise entre 250 et 550 °C.

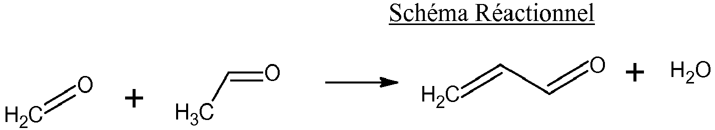

Dans les conditions de synthèse d'autres réactions peuvent exceptionnellement se produire selon les molécules (impuretés) présentes dans le milieu telles que par exemple : Production de crotonaldéhyde (2-buténal)

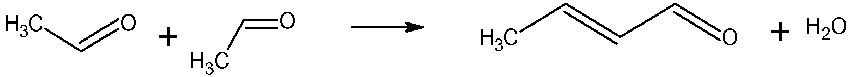

Production de méthacroléine et de penténal.

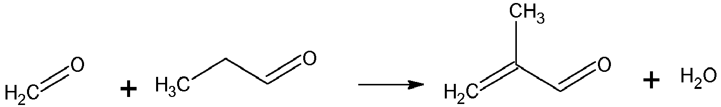

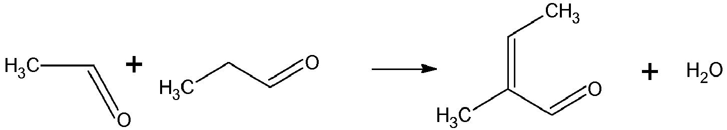

Cependant, on peut éviter la formation de ces composés penténal, obtenu par aldolisation croisée de l'acétaldéhyde et de propanaldéhyde, et méthacroléine en veillant à ce que le propanaldéhyde ne soit pas présent avec l'acétaldéhyde dans la fraction d'aldéhydes légers.

Par contre la production de pentaérythritol doit être considérée dans le schéma de procédé. Pour limiter la formation de ce produit lourd, le ratio formaldéhyde/acétaldéhyde doit être de préférence gardé inférieur à 1. De préférence, on opérera donc la réaction à conversion totale du formaldéhyde et à conversion partielle de l'acétaldéhyde..

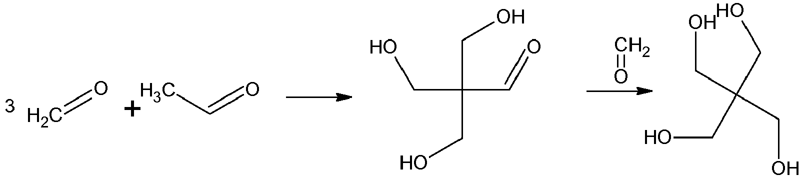

La composition du mélange entrant au réacteur de synthèse de l'acroléine par aldolisation détermine en partie les ratios molaires dans lesquels les différents produits synthétisés-acroléine et crotonoaldéhyde (2-buténaldéhyde) sont obtenus.

A la sortie du réacteur d'aldolisation, l'effluent gazeux contenant l'acroléine est refroidi et recyclé vers l'étape 2 de condensation. Ce flux peut être introduit comme charge complémentaire à l'entrée de la zone de condensation de l'étape 2 où il subit tous les traitements de la chaîne principale de purification de l'acroléine produite par déshydratation du glycérol. Il peut être également introduit dans un point intermédiaire de la zone de condensation de l'étape 2, après élimination des incondensables. Enfin, il peut être introduit à l'entrée de la zone de fractionnement de l'étape 2.

Cette forme de réalisation du procédé de l'invention présente par rapport à un procédé produisant simplement d'acroléine par déshydratation de glycérol le grand avantage de consommer moins de glycérol pour la même production d'acroléine, de générer moins de déchets puisqu'une partie d'entre eux sont consommés pour produire plus d'acroléine. En effet, lors de l'étape de déshydratation, la principale « impureté » habituelle du procédé de déshydratation est l'acétaldéhyde présent souvent au niveau de 2 à 10 % molaire, bien souvent associée à une production de formaldéhyde dans des proportions substantielles de l'ordre de 0,1 à 2. En outre on peut observer que l'acroléine produit par aldolisation est habituellement soumis à un train de purifications pour atteindre les spécifications correspondant à son usage ultérieur. Le procédé de l'invention permet de se dispenser de cette phase grâce au recyclage au niveau de l'étape 2 de la totalité de l'effluent issu du réacteur d'aldolisation.

Dans un autre mode de réalisation du procédé de l'invention, on utilisera comme réactif de la troisième étape un formaldéhyde bio-ressourcé obtenu à partir de bio-méthanol. Cette transformation peut être obtenue selon deux modes réactionnels légèrement différents. Le premier consiste en une oxydation selon la réaction suivante.

2 CH₃OH + O₂ → 2HCHO + 2H₂O

Cette réaction est conduite en phase gazeuse à une température comprise entre 200°C et 500 °C sous une pression comprise entre 1 et 5 bars absolus, généralement une pression sensiblement atmosphérique, et en présence d'un catalyseur solide choisi parmi le molybdate de fer, le tungstate de fer, les oxydes mixtes de Molybdène et d'au moins un métal W, V, Cu, Nb, Ti, ...

Dans l'autre mode réactionnel le formaldéhyde est obtenu par oxydéshydrogénation du méthanol selon la réaction suivante :

CH₃OH → HCHO + H₂

Cette réaction est conduite en phase gazeuse à une température comprise entre 500°C et 700 °C en présence d'un catalyseur à l'argent ou au cuivre métal sous une pression sensiblement atmosphérique.

Il est possible de synthétiser le formaldéhyde selon un procédé combinant les deux modes réactionnels selon la combinaison des réactions suivantes :

CH₃OH → HCHO + H₂

2 CH₃OH + O₂ → 2 HCHO + 2 H₂O

2 H₂ + O₂ → 2 H₂O

Cette combinaison de réactions est conduite en phase gazeuse à une température comprise entre 400°C et 700 °C sous une pression comprise entre 1 et 5 bars absolus et en présence d'un catalyseur solide choisi parmi le cuivre métal, l'argent métal, l'argent métal supporté sur différents supports dont le carbure de silicium, la silice, l'alumine, l'oxyde de titane.

Ces diverses réactions sont décrites dans l'Encyclopédie Ullmann volume A11 pages 624 à 631 ainsi que dans l'ouvrage de l'Institut Français du Pétrole « Procédés de pétrochimie » Tome 1, pages 105 à 114.

Dans le cas où l'on doit introduire dans le réacteur d'aldolisation de l'acétaldéhyde frais bio-ressourcé, celui-ci pourra être synthétisé par le canal de réactions analogues mettant en oeuvre le bio-éthanol telles que décrites dans l'Encyclopédie Ullmann volume A1 pages 34-35 et dans l'ouvrage de l'Institut Français du Pétrole « Procédés de pétrochimie » Tome 2 édition de 1986, pages 33 à 36.

On peut utiliser le formaldéhyde sous forme de solution aqueuse ou sous forme anhydre, par exemple le paraformaldéhyde ou le trioxanne. Dans ces derniers cas, le dérivé de formaldéhyde solide est vaporisé sous flux de gaz par chauffage.

L'acroléine obtenue selon ce procédé présente un ratio ¹⁴C /¹²C généralement compris entre 0,9 et 1,2 10⁻¹² selon que l'on utilise ou non un formaldéhyde et/ou acétaldéhyde frais non bio-ressourcé. L'acroléine obtenue présentera de préférence un ratio compris entre 1,1 et 1,2 10⁻¹².

Le procédé de l'invention sera mieux compris à la lumière de la description ci-après faite en référence aux figures annexées.
- La figure 1 illustre les étapes 1 (déshydratation du glycérol) et 2 (condensation et séparation des produits) du procédé visant la synthèse de l'acroléine suivie des étapes de purification par élimination des composés lourds et des incondensables.
- La figure 2 illustre une variante du même procédé.
- La figure 3 illustre les étapes 1 et 2 du procédé avec extraction séparée de l'acétaldéhyde et de l'acroléine dans une seule et même colonne.
- La figure 4 illustre le schéma d'un procédé complet avec ses trois étapes de synthèse de l'acroléine y compris par aldolisation de l'acétaldéhyde et du formaldéhyde issus de la phase de fractionnement de l'étape 2 et recyclage de l'effluent du réacteur d'aldolisation en tête de la zone de condensation.
- La figure 5 illustre une variante du procédé selon laquelle le recyclage par la ligne 12 s'effectue au sein de la zone de condensation en aval de la colonne d'élimination des lourds.
- La figure 6 illustre une variante du procédé selon laquelle le recyclage par la ligne 12 s'effectue à l'entrée de la zone de fractionnement.
- La figure 7 illustre une variante du procédé mettant en oeuvre le schéma de la figure 4 avec cependant une zone de fractionnement à une seule colonne comme illustré à la figure 3.

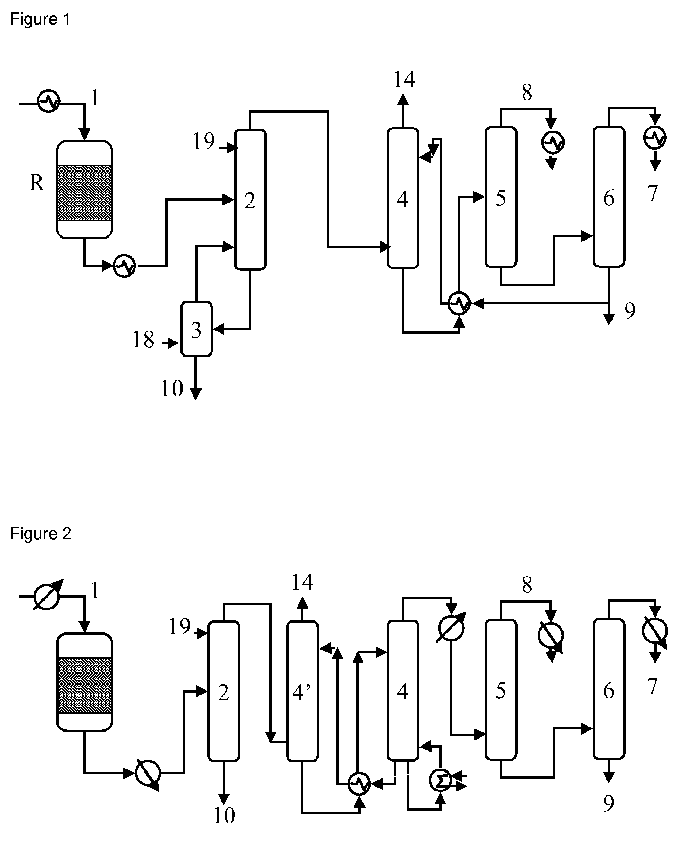

Selon la figure 1, on introduit dans le réacteur R, contenant le catalyseur acide, par la ligne 1 la charge de glycérol en mélange avec de la vapeur d'eau, après préchauffage, ainsi que de l'oxygène et un gaz inerte, tel que de l'azote. On pourra en cours d'opération substituer tout ou partie de l'azote par les gaz incondensables extraits de la colonne 4 par la ligne 14. Le réacteur est maintenu en température entre 250 et 350°C et sous une pression de 1 à 5 bars. A la sortie du réacteur on refroidit l'effluent gazeux en amont de la colonne 2 de condensation. Dans la colonne 2 où on procède à un lavage à l'eau introduite par la ligne 19, la fraction lourde est soutirée en pied et est adressée à une colonne 3 où elle est soumise à un strippage dont le but est de récupérer les aldéhydes légers qui ont été entraînés dans le flux des lourds contenant les composés acides et lourds. Ce strippage des composés les plus légers peut éventuellement être facilité par l'utilisation d'un gaz de strippage, introduit par la ligne 18 qui peut être de l'air, de l'air dilué ou des gaz de recyclage comme par exemple ceux obtenus en tête de la colonne 4. L'air (O₂) peut en outre inhiber des réactions de polymérisation dans les colonnes sous réserve d'en utiliser une quantité limitée telle qu'elle n'entraîne pas des conditions inflammables. La fraction légère est extraite en tête de colonne 3 et recyclée en bas de colonne 2. La fraction légère de la colonne 2 est sortie en tête de colonne et adressée à une colonne 4 d'élimination des gaz incondensables par strippage à la suite de l'absorption des aldéhydes dans la solution aqueuse. Dans cette colonne 4 on procède à l'extraction des composés incondensables en tête de colonne et l'effluent liquide soutiré en queue de colonne est adressé, après réchauffement par échange thermique, à une colonne 5. En tête de colonne 5 on extrait par la ligne 8 un flux riche en acétaldéhyde (et composés légers) et en queue de cette colonne on soutire un flux aqueux enrichi en acroléine qui est adressé en colonne 6. En tête de la colonne 6 on extrait par la ligne 7 le flux gazeux riche en acroléine et l'effluent de queue riche en eau, dont une fraction est purgée par extraction, et le principal est recyclé par la ligne 9 vers la colonne 4.

Le schéma de la figure 2 illustre une variante de mise en oeuvre de la partie amont, étapes 1 et 2, du procédé, déshydratation du glycérol et fractionnement des produits. A la différence du schéma de la figure 1, la colonne 2 sert au lavage à l'eau, introduite selon la flèche 19, de l'effluent issu du réacteur, les composés lourds sont soutirés en pied de cette colonne 2 par la ligne 10. La fraction gazeuse est envoyée dans une colonne 4' dont on extrait en tête les incondensables par la ligne 14, cette colonne 4' est couplée avec la colonne 4 dont l'effluent de queue est recyclé après réchauffement en haut de la colonne 4'. Les autres éléments du schéma sont identiques à ceux de la figure 1 à la seule différence que le recyclage du solvant aqueux de la ligne 9 n'est pas indiqué.

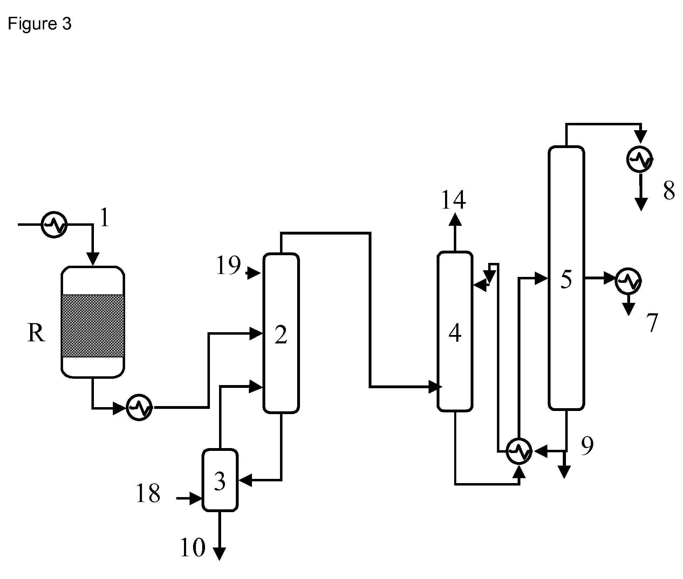

Le schéma de la figure 3 est analogue à celui de la figure 1 à la différence que le fonctionnement de la colonne 5 est modifié, ladite colonne étant pourvue d'un soutirage latéral permettant d'extraire simultanément par la ligne 7, localisée à un niveau intermédiaire, le flux gazeux riche en acroléine et en tête l'acétaldéhyde et les légers par la ligne 8. L'effluent de queue riche en eau dont une fraction est purgée par extraction et le principal recyclé par la ligne 9 vers la colonne 4

Il est à noter que certaines séparations par distillation peuvent nécessiter une élévation du niveau thermique ; dans ce cas on pourra implanter un rebouilleur à la base d'une colonne nécessitant cette élévation. Pour simplifier la présentation ce rebouilleur et les autres unités annexes ne sont pas systématiquement indiqués dans les schémas.

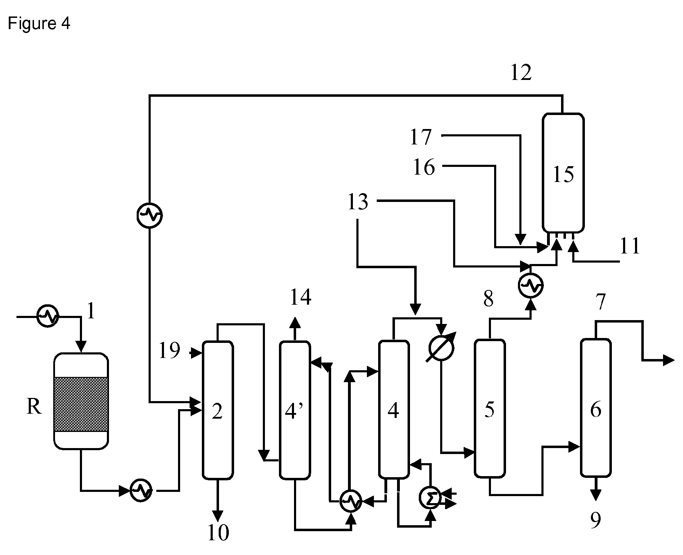

La figure 4 représente le schéma de l'ensemble d'un procédé avec ses trois étapes. Il est identique au schéma de la figure 2 pour toute la partie amont du procédé, du réacteur R à la colonne 6. En aval des colonnes 5 et 6, le flux 8, réchauffé, est adressé au réacteur 15 pour réaliser la synthèse de l'acroléine par aldolisation de l'acétaldéhyde contenu dans le flux 8 et éventuellement celui introduit par la ligne 13, avec le formaldéhyde. Par une ligne 11, on introduit éventuellement un complément de formaldéhyde. On introduit dans le réacteur 15 par la ligne 16 de l'air (ou une source d'oxygène) ainsi que de l'azote (ligne17). Ce réacteur, fonctionnant en phase gaz à une température comprise généralement entre 260 et 350°C , sous une pression généralement comprise entre 0,8 et 2 bars et une V.V.H. comprise entre de 100 et 2500 h⁻¹, est pourvu du catalyseur solide de réaction de condensation. L'effluent gazeux contenant l'acroléine produite, les réactifs non convertis et les autres coproduits dont l'eau est extrait par la ligne 12 et adressé après refroidissement à la colonne 2. La quantité de formaldéhyde et/ou d'acétaldéhyde nécessaire à la réaction non fournie par la ligne 8 est complétée par une quantité additionnelle par le canal de la ligne 13 ou la ligne 11.

Dans une variante préférée de mise oeuvre du procédé par exemple décrit en référence à la figure 4 on pourra faire fonctionner une unité de production d'acroléine sans apport externe d'acétaldéhyde. En effet, l'effluent riche en acroléine issu de la colonne 6 est sorti par la ligne 7 servant à la fabrication de l'acroléine en tant que produit. Le flux sortant par la ligne 8 contient non seulement de l'acétaldéhyde, mais dans le cas de la conversion du glycérol en acroléine, ce flux riche en acétaldéhyde contient aussi du formaldéhyde. Il suffit de choisir le débit des flux de la ligne 11 afin que le complément corresponde à une quantité de formaldéhyde telle que combinée avec les teneurs en constituants et les débits d'alimentation du courant 8 dans le réacteur 15, on ait les ratios molaires choisis pour la réaction d'aldolisation, et cela en s'affranchissant éventuellement de l'alimentation complémentaire via la ligne 13..

De façon analogue, dans le cas où le courant 8 aurait une teneur en formaldéhyde telle que le ratio molaire formaldéhyde/acétaldéhyde serait supérieur à celui retenu pour la réaction d'aldolisation, le complément en acétaldéhyde serait apporté par la ligne 13.

Sur l'unité industrielle, les divers éléments la constituant, la taille des réacteurs notamment, seront définis initialement sur la base d'un fonctionnement « normal » (nominal) en termes de capacités. Dans le cas où le réacteur R serait en « déficit » de fonctionnement par rapport à sa capacité ou par exemple en régénération, une alimentation complémentaire en formaldéhyde via 11 et en acétaldéhyde via 13 pourra permettre un fonctionnement optimal du réacteur 15 et par conséquent du train de distillation.

Dans cette configuration du schéma de procédé il n'est pas nécessaire que la séparation dans la colonne 5 de l'acroléine et de l'acétaldéhyde soit parfaite. La colonne 5 peut être réglée de telle façon qu'elle laisse passer dans le flux 8 tout l'acétaldéhyde et de ce fait également une certaine quantité d'acroléine en tête avec d'autres aldéhydes et cétones légers. On entend par aldéhydes et cétones légers ceux qui ont des points d'ébullition inférieurs ou égaux à l'acroléine tels que aldéhydes et cétones légers En effet, il est préférable de laisser passer de l'acroléine dans l'acétaldéhyde, et d'envoyer à la séparation dans la colonne 6 un flux plus pauvre en acétaldéhyde, mais en même temps ayant un débit plus faible, sans devoir excessivement grossir la colonne 5, ce qui permet de réduire les investissements nécessaires et de respecter plus facilement les critères de qualité de l'acroléine.

Dans cette configuration par la combinaison d'une production d'acroléine par déshydratation du glycérol et d'une autre production d'acroléine par aldolisation de l'acétaldéhyde par le formaldéhyde les flux sont mieux valorisés ce qui par conséquent facilite l'application industrielle.

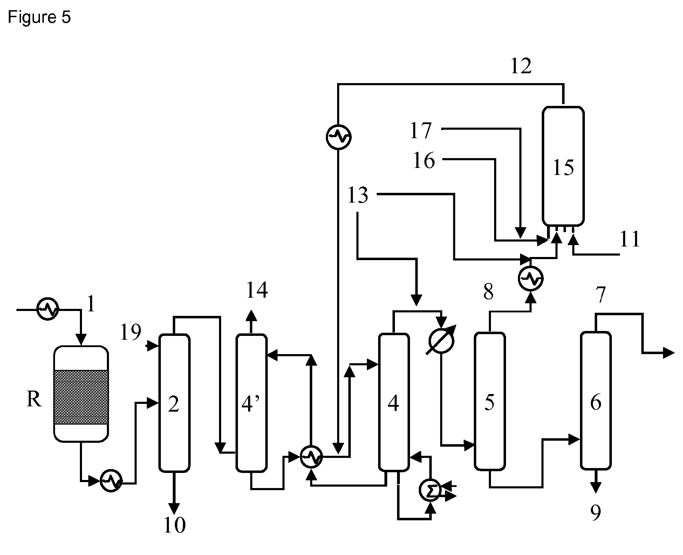

La figure 5 illustre une variante du procédé selon laquelle l'acroléine synthétisée par réaction d'aldolisation entre l'acétaldéhyde et le formaldéhyde, est adressé par la ligne 12 après refroidissement à la zone de condensation en aval de la colonne 4' et/ou en amont de la colonne 4. Selon ce schéma de procédé le flux 12 est renvoyé dans le flux allant de la colonne 4' à la colonne 4, et sert au lavage des gaz incondensables. L'acétaldéhyde et l'acroléine seront absorbés et éliminés en tête de colonne 4, les lourds produits seront retournés avec l'eau vers la colonne 4'. L'avantage de cette configuration consiste en ce que le flux chaud venant de 12, permet de réchauffer le flux allant de la colonne 4' à la colonne 4. Dans l'hypothèse d'une alimentation complémentaire en acétaldéhyde, celle-ci pourra s'effectuer soit au niveau de la ligne 8 soit en amont de la colonne 5 où s'effectue la séparation des aldéhydes légers et de l'acroléine.

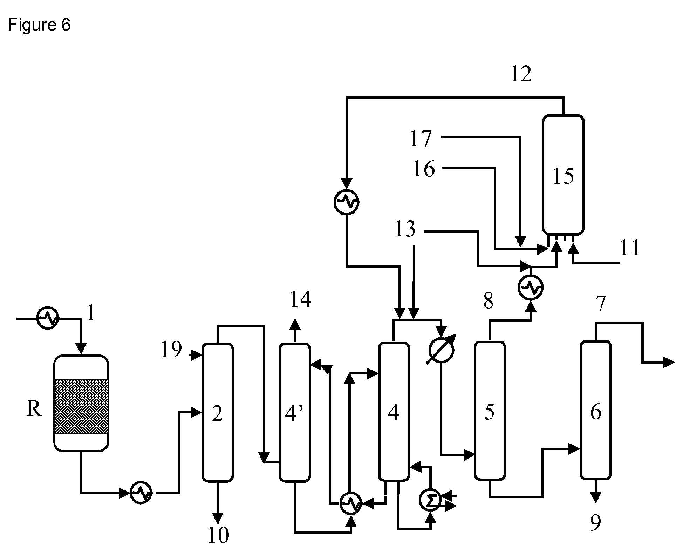

La figure 6 illustre une variante du procédé selon laquelle l'acroléine synthétisée par réaction d'aldolisation entre l'acétaldéhyde et le formaldéhyde, est adressé par la ligne 12 après refroidissement à l'entrée de la zone de fractionnement dans le courant allant de la colonne 4 à la colonne 5.

Selon cette configuration du procédé le flux 12 contenant principalement de l'acroléine, de l'acétaldéhyde et de l'eau, ces derniers sont envoyés dans la colonne 5 où l'acétaldéhyde qui n'a pas été convertie est distillée à nouveau. Si la conversion dans le réacteur 15 est partielle, alors de l'acétaldéhyde s'accumule dans cette boucle, jusqu'à atteindre une concentration d'équilibre, telle que la quantité d'acétaldéhyde produite dans le réacteur R est approximativement égale à celle convertie dans le réacteur 15. Les éventuels produits lourds formés dans le réacteur 15 sont éliminés en 9.

Dans ces configurations le propanaldéhyde est éliminé avec l'acroléine et n'est donc pas envoyé avec l'acétaldéhyde vers le réacteur 15. On ne forme donc pas de méthacroléine dans ce procédé et il n'est donc pas nécessaire de chercher à la séparer.

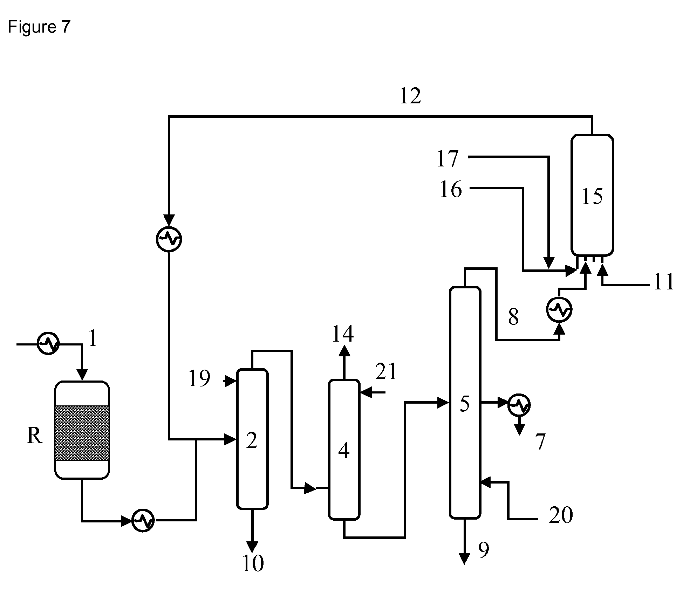

La figure 7 illustre une variante du procédé mettant en oeuvre le schéma de la figure 4 avec une zone de fractionnement simplifiée à une seule colonne analogue à celle de la figure 3. Dans cette variante, sans recyclage de l'effluent aqueux de la ligne 9, on injecte en bas de colonne 5 via la ligne 20 de la vapeur d'eau sous une pression de 4 bars et on injecte et tête de colonne 4 de l'eau d'absorption par la ligne 21.

Le procédé de l'invention est illustré par les exemples suivants.
L'exemple 1 vise la synthèse de l'acroléine selon le procédé « conventionnel » tel qu'illustré par la figure 2. Il s'agit d'un exemple comparatif.
L'exemple 2 illustre le procédé de l'invention tel qu'illustré par la figure 4.
Les exemples 3 à 6 décrivent le procédé mis en oeuvre selon le schéma décrit à la figure 7 en faisant varier les conditions opératoires du réacteur d'aldolisation: quantité de formaldéhyde, ratio formaldéhyde/acétaldéhyde, température, V.V.H.

### Exemples 1 (comparatif) et 2

Les conditions opératoires du réacteur R mises en oeuvre à l'identique dans les deux exemples sont les suivantes : Température : 320 °C ; Pression : 2,8 bars absolus ; Catalyseur : Zircone tungstée (ref Z1044 de Dai Ichi Kigenso) ; WH de 2250 h⁻¹.

Les conditions opératoires du réacteur 15 mises en oeuvre dans l'exemple 2 sont les suivantes : Température : 300 °C ; Pression : 1,3 bars ; Catalyseur : Silicate de sodium supporté sur silice.

Les mesures de débits molaires au sein des éléments principaux du procédé sont données dans les tableaux 1 (exemple comparatif) et 2 (selon l'invention) ci-dessous.

**Tableau 1**

| | **1** | **Sortie R** | **Entrée 4** | **Entrée 5** | **Entrée 6** | **8** | **7** |
|---|---|---|---|---|---|---|---|
| **Débit molaire kmol/h** | | | | | | | |
| GLYCEROL | 3,274 | 0,033 | 0,000 | 0,000 | 0,000 | 0,000 | 0,000 |
| EAU | 41,112 | 47,661 | 105,059 | 0,319 | 0,319 | 0,000 | 0,105 |
| O2 | 2,972 | 2,467 | 0,074 | 0,005 | 0,000 | 0,005 | 0,000 |
| ARGON | 0,062 | 0,062 | 0,002 | 0,000 | 0,000 | 0,000 | 0,000 |
| ACROLEINE | 0,001 | 2,620 | 2,580 | 2,511 | 2,511 | 0,000 | 2,511 |
| CO2 | 7,139 | 7,336 | 0,954 | 0,210 | 0,000 | 0,210 | 0,000 |
| ACETALDEHYDE | 0,000 | 0,295 | 0,286 | 0,265 | 0,000 | 0,265 | 0,000 |
| ACETONE | 0,000 | 0,003 | 0,003 | 0,003 | 0,003 | 0,000 | 0,003 |
| PROPIONALDEHYDE | 0,000 | 0,016 | 0,005 | 0,005 | 0,005 | 0,000 | 0,005 |
| ACETAL de GLYCEROL | 0,000 | 0,008 | 0,000 | 0,000 | 0,000 | 0,000 | 0,000 |
| ACIDE ACRYLIQUE | 0,000 | 0,033 | 0,000 | 0,000 | 0,000 | 0,000 | 0,000 |
| CO | 0,003 | 0,393 | 0,012 | 0,000 | 0,000 | 0,000 | 0,000 |
| ACIDE ACETIQUE | 0,000 | 0,049 | 0,000 | 0,000 | 0,000 | 0,000 | 0,000 |
| ACIDE PROPIONIQUE | 0,000 | 0,003 | 0,000 | 0,000 | 0,000 | 0,000 | 0,000 |
| ALCOOL ALLYLIQUE | 0,000 | 0,007 | 0,000 | 0,000 | 0,000 | 0,000 | 0,000 |
| FORMALDEHYDE | 0,001 | 0,246 | 0,238 | 0,179 | 0,179 | 0,000 | 0,000 |
| ACIDE FORMIQUE | 0,000 | 0,010 | 0,000 | 0,000 | 0,000 | 0,000 | 0,000 |
| HYDROXYACETONE | 0,000 | 0,033 | 0,000 | 0,000 | 0,000 | 0,000 | 0,000 |
| Température | 320 °C | | 70 °C | 5 °C | | | |
| Pression | 2,8 bars | | 1,7 bars | 1,7 bars | | | |

**Tableau 2**

| | **1** | **Sortie R** | **Entrée 2** | **Entrée 4** | **Entrée 5** | **Entrée 6** | **8** | **7** | **11** | **12** |
|---|---|---|---|---|---|---|---|---|---|---|
| **Débit Molaire kmol/h** | | | | | | | | | | |
| GLYCEROL | 3,274 | 0,033 | 0,033 | 0,000 | 0,000 | 0,000 | 0,000 | 0,000 | 0,000 | 0,000 |
| EAU | 41,112 | 47,661 | 49,009 | 106,054 | 0,344 | 0,344 | 0,000 | 0,113 | 1,145 | 1,348 |
| O2 | 2,972 | 2,467 | 2,467 | 0,074 | 0,005 | 0,000 | 0,005 | 0,000 | 0,000 | 0,000 |
| ARGON | 0,062 | 0,062 | 0,062 | 0,002 | 0,000 | 0,000 | 0,000 | 0,000 | 0,000 | 0,000 |
| ACROLEINE | 0,001 | 2,620 | 2,823 | 2,780 | 2,705 | 2,705 | 0,000 | 2,705 | 0,000 | 0,203 |
| CO2 | 7,139 | 7,336 | 7,336 | 0,954 | 0,210 | 0,000 | 0,210 | 0,000 | 0,000 | 0,000 |
| ACETALDEHYDE | 0,000 | 0,295 | 0,404 | 0,391 | 0,363 | 0,000 | 0,363 | 0,000 | 0,000 | 0,109 |
| ACETONE | 0,000 | 0,003 | 0,003 | 0,003 | 0,003 | 0,003 | 0,000 | 0,003 | 0,000 | 0,000 |
| PROPIONALDEHYD E | 0,000 | 0,016 | 0,016 | 0,005 | 0,005 | 0,005 | 0,000 | 0,005 | 0,000 | 0,000 |
| ACETAL de GLYCEROL | 0,000 | 0,008 | 0,008 | 0,000 | 0,000 | 0,000 | 0,000 | 0,000 | 0,000 | 0,000 |
| ACIDE ACRYLIQUE | 0,000 | 0,033 | 0,033 | 0,000 | 0,000 | 0,000 | 0,000 | 0,000 | 0,000 | 0,000 |
| CO | 0,003 | 0,393 | 0,393 | 0,012 | 0,000 | 0,000 | 0,000 | 0,000 | 0,000 | 0,000 |
| ACIDE ACETIQUE | 0,000 | 0,049 | 0,049 | 0,000 | 0,000 | 0,000 | 0,000 | 0,000 | 0,000 | 0,000 |
| ACIDE PROPIONIQUE | 0,000 | 0,003 | 0,003 | 0,000 | 0,000 | 0,000 | 0,000 | 0,000 | 0,000 | 0,000 |
| ALCOOL ALLYLIQUE | 0,000 | 0,007 | 0,007 | 0,000 | 0,000 | 0,000 | 0,000 | 0,000 | 0,000 | 0,000 |
| FORMALDEHYDE | 0,001 | 0,246 | 0,246 | 0,238 | 0,179 | 0,179 | 0,000 | 0,000 | 0,254 | 0,000 |
| ACIDE FORMIQUE | 0,000 | 0,010 | 0,010 | 0,000 | 0,000 | 0,000 | 0,000 | 0,000 | 0,000 | 0,000 |
| HYDROXYACETON E | 0,000 | 0,033 | 0,033 | 0,000 | 0,000 | 0,000 | 0,000 | 0,000 | 0,000 | 0,000 |
| Température | 320°C | | | 70 °C | 5 °C | | | | | |
| Pression | 2,8bars | | | 1,7 bars | 1,7 bars | | | | | |

### Exemples 3 à 6

Les exemples 3 à 6 sont conduits sur la base du schéma de procédé de la figure 7. Par souci de simplicité, certaines pièces d'équipement (compresseurs, rebouilleurs, condenseurs, purges...) sont volontairement omises.

Les conditions opératoires du réacteur R de déshydratation du glycérol en acroléine sont pour ces différents exemples globalement analogues à celles des exemples 1 et 2. Les différences portent sur les conditions opératoires du réacteur d'aldolisation 15.

Dans l'exemple 3 on utilise pour alimenter le réacteur 15 une solution aqueuse de formaldéhyde à 27 % poids et un ratio molaire Formaldéhyde/Acétaldéhyde de 0,7. La réaction est conduite à une température de 300 °C sous une VVH de 500 h⁻¹ en présence du catalyseur de condensation utilisé dans l'exemple 2. Une analyse détaillée des teneurs des divers flux en acroléine et divers composés est donnée dans le tableau 3 ; on peut observer que l'on obtient ainsi une augmentation de près de 10 % de la productivité en acroléine par rapport à la teneur en acroléine disponible à la sortie du réacteur R.

Dans l'exemple 4 on utilise pour alimenter le réacteur 15 une solution aqueuse de formaldéhyde à 27 % poids et un ratio molaire Formaldéhyde/Acétaldéhyde de 0,8 La réaction est conduite à une température de 300 °C sous une VVH de 300 h⁻¹ en présence du catalyseur de condensation utilisé dans l'exemple 2. Une analyse détaillée des teneurs des divers flux en acroléine et divers composés est donnée dans le tableau 4 ; on peut observer que l'on obtient ainsi une augmentation de près de 10 % de la productivité en acroléine.

Dans l'exemple 5 on utilise pour alimenter le réacteur 15 du paraformaldéhyde qui est sublimé sous un flux d'air dilué et un ratio molaire Formaldéhyde/Acétaldéhyde de 0,9 La réaction est conduite à une température de 325 °C sous une VVH de 750 h⁻¹ en présence du catalyseur de condensation utilisé dans l'exemple 2. Une analyse détaillée des teneurs des divers flux en acroléine et divers composés est donnée dans le tableau 5 ; on peut observer que l'on obtient ainsi une augmentation de près de 10 % de la productivité en acroléine. Dans cet exemple, des traces de crotonaldéhyde sont produites et éliminées en pied de colonne 5.

Dans l'exemple 6 on utilise pour alimenter le réacteur 15 une solution de formaldéhyde à 50 % pds et un ratio molaire Formaldéhyde/Acétaldéhyde de 0,8. On ajoute au réacteur un petit débit d'air avec la solution de formaldéhyde. La réaction est conduite à une température de 350 °C sous une VVH de 300 h⁻¹ en présence du catalyseur de condensation utilisé dans l'exemple 2. Une analyse détaillée des teneurs des divers flux en acroléine et divers composés est donnée dans le tableau 6 ; on peut observer que l'on obtient ainsi une augmentation de près de 10 % de la productivité en acroléine.

**Tableau 3**

| Exemple 3 | Réacteur 15 | | | Colonne 2 | | | | Colonne 4 | | | Colonne 5 | | | | |
|---|---|---|---|---|---|---|---|---|---|---|---|---|---|---|---|
| Flux | 8 | 11 | 12 | Sortie R | 12 | Tête de 2 | 10 | 21 | Pied de 4 | 14 | 20 | Entrée 5 | 9 | Pertes | 7 |
| | Acétaldéhyde | Formol | Produits | Produits Conv glycérol | Produits aldolisation | Tête | Résidus | Eau absorption | Sortie liquide | Sortie gaz | Vapeur 4b | Entrée liquide | Résidus | Tête | Prod acroléine |
| | | | | | | | | | | | | | | | |
| Température (°C) | 31.2 | 25.0 | 300.1 | 204.4 | 300.1 | 117.9 | 171.3 | 10.0 | 82.8 | 10.0 | 195.0 | 82.8 | 111.5 | 31.1 | 62.7 |
| Pression (bar) | 3.00 | 3.00 | 3.00 | 1.90 | 3.00 | 1.85 | 1.85 | 2.00 | 1.70 | 1.70 | 5.00 | 1.70 | 1.50 | 1.50 | 1.50 |
| Débit massique kg/h | | | | | | | | | | | | | | | |
| GLYCEROL | | | | 28.10 | | 0.113 | 27.99 | | 0.113 | | | 0.113 | 0.113 | | |
| ACROLEINE | 0.003 | | 10.93 | 111.16 | 10.93 | 122.01 | 0.083 | | 122.01 | 0.003 | | 122.01 | | | 122.01 |
| EAU | | 19.78 | 24.00 | 690.75 | 24.00 | 711.72 | 3.03 | 850.00 | 1560.99 | 0.733 | 230.35 | 1560.99 | 1786.2 | | 5.08 |
| CO | | | 0.239 | 9.27 | 0.239 | 9.51 | | | | 9.51 | | | | | |
| CO2 | 0.004 | | 0.004 | 7.27 | 0.004 | 7.27 | 0.001 | | 0.005 | 7.27 | | 0.005 | | 0.001 | |
| HYDROXYACETONE | | | | 1.03 | | 1.02 | 0.009 | | 1.02 | | | 1.02 | 1.02 | | |
| FORMALDEHYDE | | 7.32 | 0.366 | 5.36 | 0.366 | 5.33 | 0.395 | | 5.33 | | | 5.33 | 5.33 | | |
| ACETALDEHYDE | 15.34 | | 4.60 | 12.75 | 4.60 | 17.34 | 0.006 | | 17.34 | | | 17.34 | | 0.150 | 1.86 |
| PROPANALDEHYDE | | | | 0.960 | | 0.959 | | | 0.845 | 0.114 | | 0.845 | | | 0.845 |
| ACETONE | | | | 0.160 | | 0.160 | | | 0.160 | | | 0.160 | | | 0.160 |
| OXYGENE | | | | 20.44 | | 20.44 | | | | 20.44 | | | | | |
| AZOTE | | | | 121.89 | | 121.89 | | | 0.003 | 121.89 | | 0.003 | | 0.003 | |

**Tableau 4**

| Exemple 4 | Réacteur 15 | | | Colonne 2 | | | | Colonne 4 | | | Colonne 5 | | | | |
|---|---|---|---|---|---|---|---|---|---|---|---|---|---|---|---|
| Flux | 8 | 11 | 12 | Sortie R | 12 | Tête de 2 | 10 | 21 | Pied de 4 | 14 | 20 | Entrée 5 | 9 | Pertes | 7 |
| | Acétaldéhyde | Formol | Produits | Produits Conv glycérol | Produits aldolisation | Tête | Résidus | Eau absorption | Sortie liquide | Sortie gaz | Vapeur 4b | Entrée liquide | Résidus | Tête | Prod acroléine |
| | | | | | | | | | | | | | | | |
| Température (°C) | 31.2 | 25.0 | 300.1 | 204.4 | 300.1 | 118.0 | 171.5 | 10.0 | 82.7 | 10.0 | 195.0 | 82.7 | 111.5 | 31.1 | 62.7 |
| Pression (bar) | 3.00 | 3.00 | 3.00 | 1.90 | 3.00 | 1.85 | 1.85 | 2.00 | 1.70 | 1.70 | 5.00 | 1.70 | 1.50 | 1.50 | 1.50 |
| Débit massique kg/h | | | | | | | | | | | | | | | |
| GLYCEROL | | | | 28.10 | | 0.116 | 27.99 | | 0.116 | | | 0.116 | 0.116 | | |
| ACROLEINE | 0.002 | | 12.53 | 111.16 | 12.53 | 123.61 | 0.083 | | 123.61 | 0.003 | | 123.61 | | | 123.61 |
| EAU | | 22.68 | 27.20 | 690.73 | 27.20 | 714.92 | 3.01 | 850.00 | 1564.19 | 0.732 | 237.67 | 1564.19 | 1796.68 | | 5.17 |
| CO | | | 0.152 | 9.27 | 0.152 | 9.42 | | | | 9.42 | | | | | |
| CO₂ | 0.004 | | 0.004 | 7.27 | 0.004 | 7.27 | 0.001 | | 0.005 | 7.27 | | 0.005 | | 0.001 | |
| HYDROXYACETONE | | | | 1.03 | | 1.02 | 0.009 | | 1.02 | | | 1.02 | 1.02 | | |
| FORMALDEHYDE | | 8.39 | 0.419 | 5.36 | 0.419 | 5.39 | 0.394 | | 5.39 | | | 5.39 | 5.39 | | |
| ACETALDEHYDE | 15.39 | | 4.62 | 12.75 | 4.62 | 17.36 | 0.006 | | 17.36 | | | 17.36 | | 0.151 | 1.82 |
| PROPANALDEHYDE | | | | 0.960 | | 0.959 | | | 0.846 | 0.113 | | 0.846 | | | 0.846 |
| ACETONE | | | | 0.160 | | 0.160 | | | 0.160 | | | 0.160 | | | 0.160 |
| OXYGENE | | | | 20.44 | | 20.44 | | | | 20.44 | | | | | |
| AZOTE | | | | 121.89 | | 121.89 | | | 0.003 | 121.89 | | 0.003 | | 0.003 | |

**Tableau 5**

| Exemple 5 | Réacteur 15 | | | Colonne 2 | | | | Colonne 4 | | | Colonne 5 | | | | |
|---|---|---|---|---|---|---|---|---|---|---|---|---|---|---|---|
| Flux (ligne) | 8 | 11 | 12 | Sortie R | 12 | Tête de 2 | 10 | 21 | Pied de 4 | 14 | 20 | Entrée 5 | 9 | Pertes | 7 |
| | Acétaldéhyde | Formol | Produits | Produits Conv glycérol | Produits aldolisation | Tête | Résidus | Eau absorption | Sortie liquide | Sortie gaz | Vapeur 4b | Entrée liquide | Résidus | Tête | Prod acroléine |
| | | | | | | | | | | | | | | | |
| Température (°C) | 30.4 | 25.0 | 350.1 | 204.4 | 350.1 | 117.9 | 172.3 | 10.0 | 83.3 | 10.0 | 195.0 | 83.3 | 111.5 | 30.3 | 62.7 |
| Pression (bar) | 3.00 | 3.00 | 3.00 | 1.90 | 3.00 | 1.85 | 1.85 | 2.00 | 1.70 | 1.70 | 5.00 | 1.70 | 1.50 | 1.50 | 1.50 |
| Débit massique kg/h | | | | | | | | | | | | | | | |
| GLYCEROL | | | | 28.10 | | 0.126 | 27.97 | | 0.126 | | | 0.126 | 0.126 | | |
| ACROLEINE | 0.002 | | 10.90 | 111.16 | 10.90 | 121.97 | 0.082 | | 121.97 | 0.006 | | 121.97 | | | 121.97 |
| EAU | | 0.367 | 4.64 | 690.61 | 4.64 | 692.36 | 2.89 | 900.00 | 1591.53 | 0.830 | 193.11 | 1591.53 | 1779.54 | | 5.10 |
| CO | | | 0.209 | 9.27 | 0.209 | 9.47 | | | | 9.47 | | | | | |
| CO₂ | 0.004 | | 0.214 | 7.28 | 0.214 | 7.49 | 0.001 | | 0.006 | 7.48 | | 0.006 | | 0.001 | |
| HYDROXYACETONE | | | | 1.03 | | 1.02 | 0.009 | | 1.02 | | | 1.02 | 1.02 | | |
| FORMALDEHYDE | | 6.98 | 0.349 | 5.34 | 0.349 | 5.32 | 0.371 | | 5.32 | | | 5.32 | 5.32 | | |
| ACETALDEHYDE | 11.38 | | 0.569 | 12.75 | 0.569 | 13.31 | 0.004 | | 13.31 | | | 13.31 | | 0.110 | 1.82 |
| PROPANALDEHYDE | | | | 0.959 | | 0.959 | | | 0.799 | 0.160 | | 0.799 | | | 0.799 |
| CROTONALDEHYDE | | | 1.36 | | 1.36 | 1.35 | 0.003 | | 1.35 | | | 1.35 | 1.35 | | |
| ACETONE | | | | 0.160 | | 0.160 | | | 0.160 | | | 0.160 | | | 0.160 |
| OXYGENE | | 0.558 | 0.279 | 20.44 | 0.279 | 20.71 | | | 0.001 | 20.71 | | 0.001 | | | |
| AZOTE | | 20.01 | 20.01 | 121.89 | 20.01 | 141.90 | | | 0.004 | 141.90 | | 0.004 | | 0.003 | |

**Tableau 6**

| Exemple 6 | Réacteur 15 | | | Colonne 2 | | | | Colonne 4 | | | Colonne 5 | | | | |
|---|---|---|---|---|---|---|---|---|---|---|---|---|---|---|---|
| Flux | 8 | 11 | 12 | Sortie R | 12 | Tête de 2 | 10 | 21 | Pied de 4 | 14 | 20 | Entrée 5 | 9 | Pertes | 7 |
| | Acétaldéhyde | Formol | Produits | Produits Conv glycérol | Produits aldolisation | Tête | Résidus | Eau absoiption | Sortie liquide | Sortie gaz | Vapeur 4b | Entrée liquide | Résidus | Tête | Prod acroléine |
| | | | | | | | | | | | | | | | |
| Température (°C) | 30.9 | 25.0 | 350.0 | 204.4 | 350.0 | 117.9 | 171.4 | 10.0 | 83.3 | 10.0 | 195.0 | 83.3 | 111.5 | 30.8 | 62.7 |
| Pression (bar) | 3.00 | 3.00 | 3.00 | 1.90 | 3.00 | 1.85 | 1.85 | 2.00 | 1.70 | 1.70 | 5.00 | 1.70 | 1.50 | 1.50 | 1.50 |
| Débit massique kg/h | | | | | | | | | | | | | | | |
| GLYCEROL | | | | 28.10 | | 0.111 | 27.99 | | 0.111 | | | 0.111 | 0.111 | | |
| ACROLEINE | 0.001 | | 9.39 | 111.16 | 9.39 | 120.47 | 0.083 | | 120.47 | 0.002 | | 120.47 | | | 120.46 |
| EAU | | 7.34 | 11.65 | 690.74 | 11.65 | 699.37 | 3.01 | 870.00 | 1568.63 | 0.740 | 213.48 | 1568.63 | 1777.09 | | 5.03 |
| CO | | | 0.306 | 9.27 | 0.306 | 9.57 | | | | 9.57 | | | | | |
| CO₂ | 0.004 | | 0.238 | 7.28 | 0.238 | 7.52 | 0.001 | | 0.006 | 7.51 | | 0.006 | | 0.001 | |
| HYDROXYACETONE | | | | 1.03 | | 1.02 | 0.010 | | 1.02 | | | 1.02 | 1.02 | | |
| FORMALDEHYDE | | 7.34 | 0.367 | 5.37 | 0.367 | 5.33 | 0.400 | | 5.33 | | | 5.33 | 5.33 | | |
| ACETALDEHYDE | 13.46 | | 2.69 | 12.75 | 2.69 | 15.43 | 0.005 | | 15.43 | | | 15.43 | | 0.131 | 1.85 |
| PROPANALDEHYDE | | | | 0.960 | | 0.959 | | | 0.852 | 0.107 | | 0.852 | | | 0.852 |
| CROTONALDEHYDE | | | 2.14 | | 2.14 | 2.14 | 0.005 | | 2.14 | | | 2.14 | 2.14 | | |
| ACETONE | | | | 0.160 | | 0.160 | | | 0.160 | | | 0.160 | | | 0.160 |
| OXYGENE | | 0.328 | 0.016 | 20.43 | 0.016 | 20.45 | | | 0.001 | 20.45 | | 0.001 | | | |
| AZOTE | | 1.38 | 1.38 | 121.89 | 1.38 | 123.27 | | | 0.004 | 123.26 | | 0.004 | | 0.003 | |

## Revendications

1. Procédé de synthèse d'acroléine consistant
- dans une première étape à soumettre une charge de glycérol issue de la méthanolyse des huiles végétales ou des graisses animales, à une réaction de déshydratation conduisant à l'acroléine selon la réaction CH₂OH-CHOH-CH₂OH → CH₂=CH-CHO + 2H₂O puis,
- dans une deuxième étape après avoir soumis l'effluent issu de la première étape à un refroidissement à procéder tout d'abord dans une première zone à un lavage à l'eau et la condensation de l'effluent gazeux issu de la première étape, pour séparer ensuite dans une deuxième zone de fractionnement d'une première part un flux riche en aldéhydes légers, d'une deuxième part un flux riche en eau et d'une troisième part le flux d'acroléine et, puis
- dans une troisième étape à faire réagir l'acétaldéhyde contenu dans le flux riche en aldéhydes légers issu de l'étape précédente avec du formaldéhyde pour obtenir un second flux riche en acroléine par réaction d'aldolisation selon le schéma réactionnel suivant : CH₃-CHO + CH₂O → CH₂=CH-CHO + H₂O et à recycler ce second flux riche en acroléine au niveau de l'étape 2 précédente, le réacteur d'aldolisation étant alimenté par le flux d'aldéhydes légers issu de l'étape 2 qui est complété par un flux auxiliaire d'acétaldéhyde et/ou de formaldéhyde tel que le rapport molaire acétaldéhyde/ formaldéhyde au sein du réacteur est compris entre 0,3 et 1,5.

2. Procédé selon la revendication 1 **caractérisé en ce que** le réacteur d'aldolisation est alimenté par le flux d'aldéhydes légers issu de l'étape 2 qui est complété par un flux auxiliaire d'acétaldéhyde et/ou de formaldéhyde tel que le rapport molaire acétaldéhyde/ formaldéhyde au sein du réacteur est compris entre 0,5 et 1.

3. Procédé selon l'une des revendications 1 ou 2 **caractérisé en ce que** la première étape de déshydratation du glycérol est réalisée en phase gaz dans le réacteur en présence d'un catalyseur solide à une température allant de 150°C à 500°C, de préférence comprise entre 250°C et 350°C, et une pression comprise entre 1 et 5 bars.

4. Procédé selon la revendication 3 **caractérisé en ce que** cette étape est conduite en présence d'oxygène.

5. Procédé selon l'une des revendications 3 ou 4 **caractérisé en ce que** le catalyseur solide est constitué à partir de matériaux homogènes ou multiphases, insolubles dans le milieu réactionnel et qui ont une acidité de Hammett, notée H₀ inférieure à +2.

6. Procédé selon l'une quelconque des revendications 1 à 5 **caractérisé en ce que** la réaction d'aldolisation de la troisième étape est réalisée en phase gazeuse à une température comprise entre 150°C et 400 °C, de préférence entre 260°C et 350°C, à une pression généralement comprise entre 0,5 et 10 bars, de préférence entre 0,8 et 2 bars, en présence d'un catalyseur solide de condensation.

7. Procédé selon la revendication 6 **caractérisé en ce que** l'étape est conduite en présence d'oxygène.

8. Procédé selon la revendication 6 **caractérisé en ce que** le catalyseur est un solide tel que d'une part, les chaleurs moyennes d'adsorption du CO₂, du SO₂ et de NH₃ seront respectivement comprises entre 40 et 170 kJ/mole à 303 °K (CO₂), entre 25 et 180 kJ/mole à 353 °K (SO₂) et entre 25 et 170 kJ/mole à 423 °K (NH₃) et d'autre part, la quantité de NH₃ adsorbée sur la surface du solide sera comprise entre 1,5 et 8 micromole/m² et celle de CO₂ comprise entre 0,5 et 8 micromole/m².

9. Procédé selon l'une quelconque des revendications 1 à 8 **caractérisé en ce que** le second flux riche en acroléine issu de la réaction d'aldolisation est recyclé à l'entrée de la zone de condensation de l'étape 2.

10. Procédé selon l'une quelconque des revendications 1 à 8 **caractérisé en ce que** le second flux est recyclé dans un point intermédiaire de la zone de condensation de l'étape 2, après élimination des incondensables.

11. Procédé selon l'une quelconque des revendications 1 à 8 **caractérisé en ce que** le second flux est recyclé à l'entrée de la zone de fractionnement de l'étape 2.

## Patentansprüche

1. Verfahren zur Synthese von Acrolein, bei dem man
- in einem ersten Schritt eine Charge von Glycerin aus der Methanolyse von Pflanzenölen oder Tierfetten einer Dehydratisierungsreaktion unterwirft, die gemäß der Reaktion CH₂OH-CHOH-CH₂OH → CH₂=CH-CHO + 2H₂O zu Acrolein führt, dann
- in einem zweiten Schritt nach dem Abkühlen des Austragsstroms aus dem ersten Schritt zunächst in einer ersten Zone eine Wäsche mit Wasser und die Kondensation des gasförmigen Austragsstroms aus dem ersten Schritt durchführt und dann in einer zweiten Fraktionierungszone erstens einen an niederen Aldehyden reichen Strom, zweitens einen wasserreichen Strom und drittens den Acroleinstrom abtrennt und dann
- in einem dritten Schritt den in dem an niederen Aldehyden reichen Strom aus dem vorhergehenden Schritt enthaltenen Acetaldehyd mit Formaldehyd umsetzt, wodurch man durch Aldolisationsreaktion gemäß dem folgenden Reaktionsschema: CH₃-CHO + CH₂O → CH₂=CH-CHO + H₂O einen zweiten acroleinreichen Strom erhält und diesen zweiten acroleinreichen Strom zum vorhergehenden Schritt 2 zurückführt, wobei der Aldolisationsreaktor durch den Strom niederer Aldehyde aus Schritt 2 gespeist wird, welcher durch einen zusätzlichen Strom von Acetaldehyd und/oder Formaldehyd derart ergänzt wird, dass das Acetaldehyd/Formaldehyd-Molverhältnis im Reaktor zwischen 0,3 und 1,5 liegt.

2. Verfahren nach Anspruch 1, **dadurch gekennzeichnet, dass** der Aldolisationsreaktor durch den Strom niederer Aldehyde aus Schritt 2 gespeist wird, welcher durch einen zusätzlichen Strom von Acet-aldehyd und/oder Formaldehyd derart ergänzt wird, dass das Acetaldehyd/Formaldehyd-Molverhältnis im Reaktor zwischen 0,5 und 1 liegt.

3. Verfahren nach Anspruch 1 oder 2, **dadurch gekennzeichnet, dass** der erste Schritt der Dehydratisierung von Glycerin in der Gasphase in dem Reaktor in Gegenwart eines festen Katalysators bei einer Temperatur im Bereich von 150°C bis 500°C und vorzugsweise zwischen 250°C und 350°C und einem Druck zwischen 1 und 5 bar durchgeführt wird.

4. Verfahren nach Anspruch 3, **dadurch gekennzeichnet, dass** dieser Schritt in Gegenwart von Sauerstoff durchgeführt wird.

5. Verfahren nach Anspruch 3 oder 4, **dadurch gekennzeichnet, dass** der feste Katalysator aus homogenen oder mehrphasigen Materialien besteht, die im Reaktionsmedium unlöslich sind und eine Hammett-Acidität H₀ von weniger als +2 aufweisen.

6. Verfahren nach einem der Ansprüche 1 bis 5, **dadurch gekennzeichnet, dass** die Aldolisationsreaktion des dritten Schritts in der Gasphase bei einer Temperatur zwischen 150°C und 400°C und vorzugsweise zwischen 260°C und 350°C bei einem Druck, der im Allgemeinen zwischen 0,5 und 10 bar und vorzugsweise zwischen 0,8 und 2 bar liegt, in Gegenwart eines festen Kondensationskatalysators durchgeführt wird.

7. Verfahren nach Anspruch 6, **dadurch gekennzeichnet, dass** der Schritt in Gegenwart von Sauerstoff durchgeführt wird.

8. Verfahren nach Anspruch 6, **dadurch gekennzeichnet, dass** es sich bei dem Katalysator um einen solchen Feststoff handelt, dass einerseits die mittleren Adsorptionswärmen von CO₂, SO₂ and NH₃ zwischen 40 und 170 kJ/mol bei 303°K (CO₂), zwischen 25 und 180 kJ/mol bei 353°K (SO₂) bzw. zwischen 25 und 170 kJ/mol bei 423°K (NH₃) liegen und andererseits die an der Oberfläche des Feststofffs adsorbierte NH₃-Menge zwischen 1,5 und 8 Mikromol/m² und die adsorbierte CO₂-Menge zwischen 0,5 und 8 Mikromol/m² liegt.

9. Verfahren nach einem der Ansprüche 1 bis 8, **dadurch gekennzeichnet, dass** der zweite acroleinreiche Strom aus der Aldolisationsreaktion zum Eingang der Kondensationszone von Schritt 2 zurückgeführt wird.

10. Verfahren nach einem der Ansprüche 1 bis 8, **dadurch gekennzeichnet, dass** der zweite Strom nach Entfernung der nicht kondensierbaren Verbindungen zu einem intermediären Punkt der Kondensationszone von Schritt 2 zurückgeführt wird.

11. Verfahren nach einem der Ansprüche 1 bis 8, **dadurch gekennzeichnet, dass** der zweite Strom zum Eingang der Fraktionierungszone von Schritt 2 zurückgeführt wird.

## Claims

1. Process for synthesizing acrolein consisting
- in a first step, in subjecting a glycerol charge resulting from the methanolysis of plant oils or of animal fats to a dehydration reaction resulting in acrolein according to the reaction CH₂OH-CHOH-CH₂OH → CH₂=CH-CHO + 2H₂O, then,
- in a second step, after having subjected the effluent resulting from the first step to cooling, in carrying out first of all, in a first zone, washing with water and condensation of the gaseous effluent resulting from the first step, so as to then separate, in a second fractionation zone, firstly a light aldehydes-rich stream, secondly a water-rich stream and thirdly the acrolein stream, and then,
- in a third step, in reacting the acetaldehyde contained in the light aldehydes-rich stream resulting from the preceding step with formaldehyde so as to obtain an acrolein-rich second stream by aldol condensation reaction according to the following reaction scheme: CH₃-CHO + CH₂O → CH₂=CH-CHO + H₂O and in recycling this acrolein-rich second stream to the preceding step 2, the aldol condensation reactor being supplied by the stream of light aldehydes resulting from step 2, which is supplemented with an auxiliary stream of acetaldehyde and/or of formaldehyde such that the acetaldehyde/formaldehyde mole ratio in the reactor is between 0.3 and 1.5.

2. Process according to Claim 1, **characterized in that** the aldol condensation reactor is supplied by the stream of light aldehydes resulting from step 2, which is supplemented with an auxiliary stream of acetaldehyde and/or of formaldehyde such that the acetaldehyde/formaldehyde mole ratio in the reactor is between 0.5 and 1.

3. Process according to either of Claims 1 and 2, **characterized in that** the first glycerol dehydration step is carried out in the gas phase in the reactor in the presence of a solid catalyst at a temperature ranging from 150°C to 500°C, preferably of between 250°C and 350°C, and a pressure of between 1 and 5 bar.

4. Process according to Claim 3, **characterized in that** this step is carried out in the presence of oxygen.

5. Process according to either of Claims 3 and 4, **characterized in that** the solid catalyst is made up of homogeneous or multiphase materials, which are insoluble in the reaction medium and which have a Hammett acidity, denoted Ho, of less than +2.

6. Process according to any one of Claims 1 to 5, **characterized in that** the aldol condensation reaction of the third step is carried out in the gas phase at a temperature of between 150°C and 400°C, preferably between 260°C and 350°C, at a pressure of generally between 0.5 and 10 bar, preferably between 0.8 and 2 bar, in the presence of a solid condensation catalyst.

7. Process according to Claim 6, **characterized in that** the step is carried out in the presence of oxygen.

8. Process according to Claim 6, **characterized in that** the catalyst is a solid such that, firstly, the average CO₂ , SO₂ and NH₃ adsorption heats will be, respectively, between 40 and 170 kJ/mol at 303°K (CO₂), between 25 and 180 kJ/mol at 353°K (SO₂) and between 25 and 170 kJ/mol at 423°K (NH₃) and, secondly, the amount of NH₃ adsorbed on the surface of the solid will be between 1.5 and 8 micromol/m² and that of CO₂ between 0.5 and 8 micromol/m².

9. Process according to any one of Claims 1 to 8, **characterized in that** the second acrolein-rich stream resulting from the aldol condensation reaction is recycled to the input of the condensation zone of step 2.

10. Process according to any one of Claims 1 to 8, **characterized in that** the second stream is recycled to an intermediate point of the condensation zone of step 2, after removal of the noncondensable compounds.

11. process according to any one of Claims 1 to 8, **characterized in that** the second stream is recycled to the input of the fractionation zone of step 2.
